# EUROPEAN PATENT SPECIFICATION

(11) **EP 2 924 431 B1**
(45) Date of publication and mention of the grant of the patent: **30.08.2017**
(21) Application number: 12888832.8
(22) Date of filing: 22.11.2012
(51) Int. Cl.: G01N 33/38, G01F 23/292

(54) **DEVICE FOR MEASURING VOLUMETRIC CHANGES IN A SUBSTANCE**
VORRICHTUNG ZUM MESSEN VOLUMETRISCHER VERÄNDERUNGEN IN EINER SUBSTANZ
DISPOSITIF ÉQUIPÉ D'UNE FONCTION DE MESURE DES CHANGEMENTS VOLUMÉTRIQUE DANS UNE SUBSTANCE

(43) Date of publication of application: 30.09.2015
(73) Proprietor: Cementos Argos S.A., Medellin (CO); Universidad Eafit, Medellín (CO)
(72) Inventor: DIAZ, Maria Fernanda, Medellin (CO); JARAMILLO, Juan Manuel, Medellin (CO); CORREA, Carlos German, Medellin (CO)
(74) Representative: Bassil, Nicholas Charles
(86) International application number: PCT/IB2012/056605
(87) International publication number: WO 2014/080243

(56) References cited:
- CN-U- 85 200 096
- DE-A1-102005 001 945
- US-A- 2 354 847
- US-A- 4 848 900
- BOUASKER ET AL: "Chemical shrinkage of cement pastes and mortars at very early age: Effect of limestone filler and granular inclusions", CEMENT AND CONCRETE COMPOSITES, ELSEVIER APPLIED SCIENCE, BARKING, GB, vol. 30, no. 1, 24 October 2007 (2007-10-24), pages 13-22, XP022313159, ISSN: 0958-9465, DOI: 10.1016/J.CEMCONCOMP.2007.06.004
- D'AMBROSIA, M. ET AL.: 'Early-Age Cracking. Concrete TechnologylEarly-age CrackinglPortland Cement Association (PCA)' 12 August 2010, XP055255106 Retrieved from the Internet: <URL:http://sab2112.blogspot.com.es/2010/08 /concrete-technology-early-age- cracking.html> [retrieved on 2013-08-12]
- KOSMATKA,S.H. ET AL.: 'Design and Control of Concrete Mixtures.' 2003, SKOKIE, ILLINOIS, USA, XP055254539 Retrieved from the Internet: <URL:http://learn.roguecc.edu/construction/ rhenderson/documents/ DesignAndControlConcrete.pdf> [retrieved on 2013-08-12]

## Description

### Technical field

This invention relates to a device with function for measuring volumetric changes in a substance, comprising a sensor that detects the amount of light intensity produced by a light source aligned with said sensor, when passing through a column of two liquids which are immiscible among each other contained in a transparent tube, wherein the sensor and the aligned light source are mounted on an arm that travels on a vertically arranged rail, wherein the arm is attached to a strap which acts by means of a first and a second pulley causing the movement of said arm, wherein the movement of said pulleys comes from a motor and a gearbox and the entire mobility system is mounted on a frame. The device further comprises data acquisition means which record the change of light intensity and control the arm movement.

### Prior Art

According to the prior art, there are disclosed several applications of the device; among them, the indirect monitoring of the chemical shrinkage of concrete associated with the hydration speed of said material, as indicated in the ASTM C1608 standard. As the concrete hydration products use less volume than the initial materials before reacting (concrete and water), the speed of said volumetric change in materials indicates the degree of chemical shrinkage the concrete has. Similarly, the art discloses some measuring instruments backgrounds in this regard, such as that disclosed in the United States document US6986279 to Lockwood et al., which discloses a device for determining the liquid absorption of an aggregate which comprises providing a stirring system, a vacuum source and a container; placing a sample of the aggregate in the container; adding enough liquid to the container so as to achieve a calibration marking in the container; weighing the sample and liquid; mounting the container in the stirring device; connecting the vacuum source to the container; stirring the sample and the liquid with the stirring device; applying a vacuum to the sample and the liquid with the vacuum source, after the stirring and vacuum steps, adding enough liquid to the container so as to achieve again the calibration marking in the container, weighing again the sample and the liquid, and subtracting the initial weight of the sample and liquid of the final weight of the sample and liquid in order to determine the liquid absorption in the aggregate. The apparatus of the art complies with the need of measuring the concrete absorption but is of high-cost construction and does not monitor the sample during the entire process, obtaining intermediate data. Further, this prior art equipment is of difficult manufacturing, handling and transportation. In "Chemical shrinkage of cement pastes and mortars at very early age: Effect of limestone filler and granular inclusions" by Bouasker, Mounanga, Turcry, Loukili, Khelidj a different technique is described, where the sample is immersed in a sealed vessel provided with a capillary. In this alternative method, the level of the capillary is followed with automatic means.

### Disclosure of the invention

### Technical Problem

The object of the present invention is to improve the shortcomings of the prior art. More particularly, the main object of the present invention is a method to measure volumetric changes in a substance as disclosed in claim 1. A related measuring device is disclosed in claim 4. Further embodiments are disclosed in the dependent claims.

### Brief Description of the Drawings

In order to further clarify the invention and the advantages thereof compared with the known art, the possible illustrative and non limitative embodiments of the application of said invention are described below with the support of the drawings.
Figure 1 shows a right side view of the device with function for measuring the volumetric changes in a substance of the present invention.
Figure 2 shows a rear view of the device with function for measuring the volumetric changes in a substance of the present invention.
Figure 3 shows a front view of the device with function for measuring the volumetric changes in a substance of the present invention.
Figure 4 shows in detail the sample collection column of the device with function for measuring the volumetric changes in a substance of the present invention.
Figure 5 shows a schematic view of the device with function for measuring the volumetric changes in a substance, including the interconnection with the systems of data recording and collection according to the present invention.

### Best way of carrying out the invention

This invention relates to a device with function for measuring volumetric changes in a substance. Particularly, the device with function for measuring volumetric changes in a substance comprises a sensor aligned to a light source which allows for measuring the change of intensity.

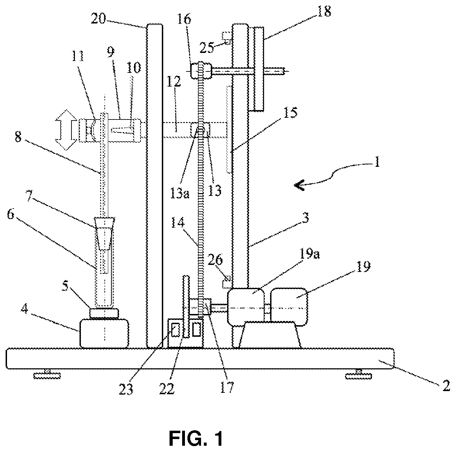

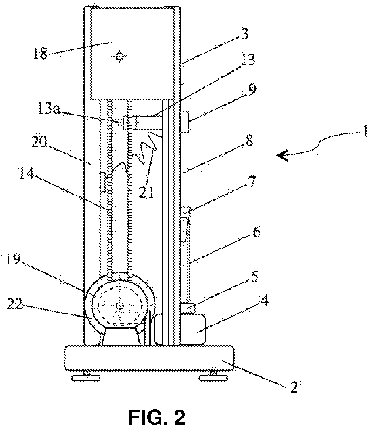

According to Figures 1, 2 and 3, the device 1 for measuring volumetric changes in a substance of the invention, comprises a base frame 2 which receives a mast frame 3 and a sample collection column 4. The sample collection column 4, in turn, comprises a receiving and positioning base 5 which supports a vessel or container 6 with top mouth opened to the environment and preferably made of glass. This vessel 6 includes a plug 7 which covers and closes said top mouth. The plug 7 further comprises a central through hole, wherein a transparent tube 8 is vertically provided and which is opened at its both ends, preferably made of glass and with graduation marks or reglette and which protrudes over the plug at least three folds the vessel 6 height, with a portion of the tube 8 entering between the vessel 6 towards the middle height of said vessel 6, traversing the plug 7. An elongated support 9 is provided aligned with the tube 8 protrusion, arranged perpendicularly with respect to the transparent tube 8, including a light source 10 and a sensor 11 facing each other, surrounding the tube 8.

In a preferred embodiment, an in an illustrative fashion, the light source 10 used for the present invention may comprise a DI650-2.5-3(5) reference laser, with a 650 nm wavelength, red colored visible spectrum, with 2 mm spot diameter and a 1.5 mrad divergence, having a 2.5 mW power output, manufactured by the company SHAANZI HUAKE OPTOELECTRONICS CO. LTD.

Likewise, the sensor 11 which allows for recording the light intensity of the light source 10 when it passes through a liquid means is an ODD-660W reference diode from the company OPTO-DIODE CORP. In an illustrative but not limitative manner, for the present invention, the sensor 11 comprises an effective 0.62 mm² area and a response centered at 650 nm of 0.42 A/W.

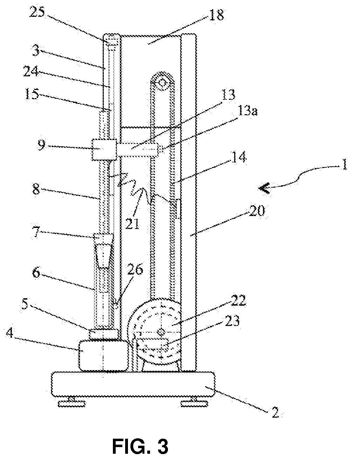

The support 9 is mounted at the end of an elongated arm 12 which protrudes horizontally; wherein said arm 12 comprises an extension 13 including a fixed connection 13a to a vertically arranged strap 14, wherein the extension 13 protrudes perpendicularly from the arm 12. To ensure the correct movement of the arm 12 towards the end opposed to the support 9, the arm 12 is attached to a lane 15 which travels along a rail 24 comprised by a channel which is vertically arranged along the mast 3 as shown in Figure 3. The lane 15 ensures the vertical movement of the arm 12 as shown in Figure 1, so as to cause the support 9 including the light source 10 and the sensor 11 to travel the free length of the tube 8.

For the vertical movement of the arm 12, the strap 14 acts by means of connection to the top 16 and bottom 17 sets of pulleys. The top pulley axis 16 is supported by a plate 18 comprising an L-fold attached to the rear top end of the frame mast 3 as shown in Figures 1, 2 and 3. For its rotation, the bottom pulley 17 is connected to a motor 19 and a gearbox 19a, which allows for the mechanical movement of the device.

In preferred embodiments, as shown in Figures 1, 2 and 3, in order to attach the connecting cables to the support 9, the device of the invention comprises a cable mast 20 which arranges and positions the connection cable 21 towards the support 9 and connects the light source 10 and the sensor 11.

The control for the movements of the arm 12 comprises an encoder 22 comprising an infrared pair 23 provided adjacent to the encoder 22, wherein said encoder 22 is coupled to the protrusion of the bottom pulley 17 axis. Said encoder 22 along with the infrared pair 23, are located for detecting the rotation and encode same so as to control the motor 19 operation and its direction of rotation.

Thus, the motor 19 provides the movement for raising and lowering the arm 12, allowing for the support 9 to travel along the entire length of the tube 8.

In the invention, at the ends of the rail 24 attached to the mast 3, there are provided the top 25 and bottom 26 sensors such that when the lane 15 along with the arm 12 reach these points, the system immediately detects the position of the lane 15 and, in such case, stops the time and height recording and returns to the initial position (end of the run at the top of the rail support).

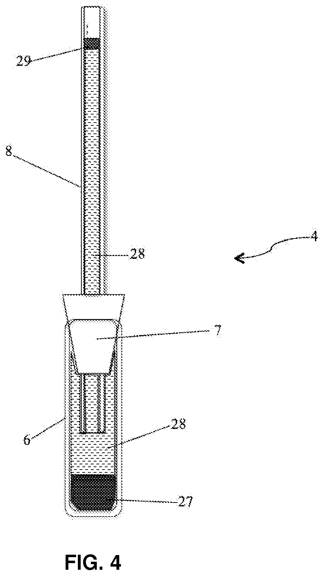

According to Figure 4, for its use in tests or trials, the sample collection column 4 contains within the vessel 6 a first volume of the solid or liquid 27 which will absorb a volume of liquid 28, a portion of which being provided within the vessel 6 and another portion within the tube 8, followed, in order to cover and close the environment, by a different colored and immiscible substance 29 of the liquid 28 to be absorbed. As the transparent tube 8 is opened on both sides, when introducing same in the vessel 6 with plug 7, the level of the liquid (e.g., water) contained in the vessel 6 will rise through the tube 8 up to a specified level. Subsequently, a small volume of an immiscible liquid (e.g., oil) and with an absorption rate significantly different from the first is added over the specified level.

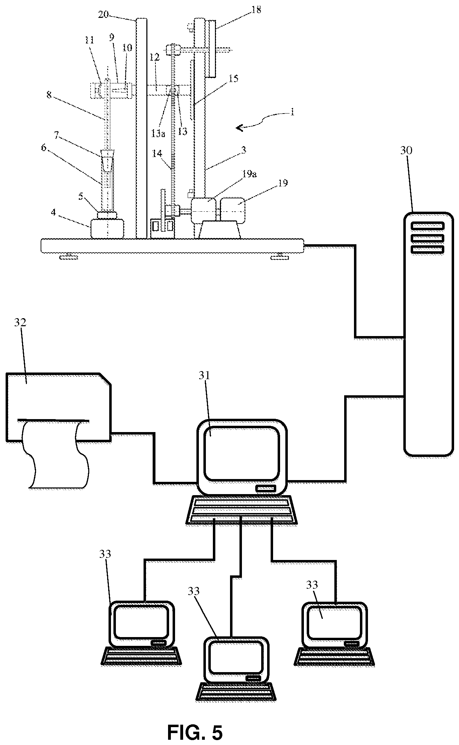

Based on Figure 5, in order to determine the record of the data emitted from the support 9 including the light source 10 and the sensor 11 both for regulating the movement of the arm 12 as well as all the variables of the trial, electronic processing means 30 are provided for establishing, through a logic support, an order of sequences to be treated. Further, the equipment comprises a computerized terminal 31 for managing such data, printing by printing means 32, and data transmission, if applicable, to remote terminals 33.

The device of the invention has been conceived such that the data are received by the software, the latter being responsible for interpreting the voltage values recorded by the sensor 11 and providing the information required by the for measuring and analysis. Then, the electronic processing means must perform the functions of recording the light intensity emitted by the light source 10 detected by the sensor 11; moving said light source and sensor along the tube 8; recording the amount of movement; and recording the time of change of means for later saving said data in an internal memory which is part of the electronic plate.

In order to perform these movement and recording functions, electronic support components such as a control unit which component is a micro-controller are provided, through which the commands are sent to the mechanical system wherein the motor 19 is started by relays.

The device of the invention further comprises a memory in which the data are stored.

Likewise, so as to ensure a correct leveling of the device 1 over the working surface, the base frame 2 may comprise levels along with height adjustable supports such as bolt-adjustable legs.

The entire system is controlled by the micro-controller, which receives instructions from a computer. After being started by the computer, the system is independent and collects the required information as per its programming.

According to Figures 1, 2 and 3, when the equipment is started, the arm 12 and the support 9 start descending and recording the light intensity crossing the air contained within the tube 8. The mechanical arm 12 starts moving vertically towards the base or bottom end of the tube 8 until it encounters the light intensity corresponding to the immiscible liquid (e.g., oil). The equipment records that height value and time equals zero as a baseline level. From this baseline level, the mechanical arm 12 descends to a specified height remaining fixed. At that point, the time starts to be recorded until the immiscible liquid (e.g., oil) reaches the specified height and the light intensity variation is produced when passing from the absorbed liquid (e.g., water) to the immiscible liquid (e.g., oil). When recording such time that the immiscible liquid took for reaching the specified height, the mechanical arm 12 once again travels vertically along the same specified distance. The height that the system records at this point is measured from the baseline level; thus, the traveled distance is equal to twice the specified height. In this new position it remains static until the immiscible liquid (e.g., oil) reaches the new height which is equal to twice the specified height. When this happens, a variation of intensity occurs when crossing the interface (absorbed liquid and immiscible liquid). When the immiscible liquid reaches the new height position, the device of the invention records the time elapsed from the baseline level until that time. Once the new height and time (cumulative) recorded, the arm travels again to the initial specified height. The height that the equipment records now is a third height and the system behaves as described above.

The device, through a data acquisition card in the electronic processing means, records the change of light intensity produced as a result of the movement of the liquid to be absorbed (e.g., water) contained in the tube 8, since the solid or liquid absorbs some amount of volume thereof and along with the movement of the volume of the liquid to be absorbed, the movement of the different colored and immiscible substance (e.g., oil) which is in the top of the column is established. When the volume of the different colored and immiscible substance (e.g. oil) reaches the light beam that comes from the light source 10, through the sensor 11, the equipment records and saves the time and height in which this change of intensity occurred when the light passed from one means to another (e.g., from water to oil).

Similarly, from the device of the present invention, it is possible to determine the amount of a substance in a solution.

## Claims

1. A method for measuring the volumetric changes in a substance which is part of a sample (27) provided within a vessel (6) with opened top mouth, wherein the mouth is closed by a plug (7) having a hole through which a transparent tube (8) with opened ends and graduation mark passes, the tube (8) having its bottom end inside the vessel (6) and its top end outside the vessel (6), the vessel (6) being filled with a translucent liquid (28) which further fills a tube length until it forms a level of the translucent liquid (28), a light emitter/receptor set (10, 11) being comprised by a light emitter (10) provided at one side of the tube (8) and a light receptor (11) at the opposite side of the tube (8), said light emitter (10) and receptor (11) being optically aligned, wherein the method comprises the step of:
Providing a marker (29) immiscible with said translucent liquid (28) and with an opacity different from the one of said translucent liquid over said level of translucent liquid (28),
Providing said light emitter/receptor set (10, 11) along the tube (8) and over said marker (29),
Moving said light emitter/receptor (10, 11) set downstream until said marker (29) interposes between said light emitter (10) and said light receptor (11), thereby determining a variation in the intensity of the light traversing the tube (8),
Defining at that point a zero "0" time and an initial position or baseline position,
Moving said light emitter/receptor set (10, 11) downstream along a specified distance until a first position, and recording at that point a height value,
Waiting for said marker (29), in a downstream direction, to interpose between said light emitter (10) and said light receptor (11) in said first position, thereby determining a new variation in the intensity of the light reaching the light receptor (11),
Recording said height value and the time the marker (29) took for interposing between the light emitter (10) and the receptor (11);
Moving said light emitter/receptor set (10, 11) downstream until a second position, and recording at that point the new height value,
Waiting for said marker (29), in a downstream direction, to interpose between said light emitter (10) and said light receptor (11), thereby determining a new variation in the intensity of the light reaching the light receptor (11),
Recording at that point said height value and the time the marker (29) took for interposing between the light emitter (10) and the receptor (11);
Moving said light emitter/receptor set (10, 11) downstream until a third position, and recording at that point a new height value,
Waiting for said marker (29), in a downstream direction, to interpose between said light emitter (10) and said light receptor (11) and, when the marker (29) is not able to interpose between the light emitter (10) and the receptor (11) in a specified time, establishing the second position as the last position;
Recording the distance between the initial position and the last position, and
Recording the time elapsed between the time zero "0" and the time accumulated until the last position.

2. A method according to claim 1, wherein said translucent liquid (28) is water and said marker (29) is a colored immiscible liquid.

3. A method according to claim 2, wherein said colored immiscible liquid is oil.

4. A device for measuring, by means of the method of anyone of claims 1 to 3, the volumetric changes in a substance which is part of a sample, the device comprising a base frame (2) which receives a mast frame (3) and a sample collection column (4); wherein the sample collection column (4) in turn comprises a receiving and placement base (5) supporting a vessel or container (6) with top mouth opened to the environment and made of transparent material; wherein said vessel (6) comprises a plug (7) which covers and closes said top mouth; wherein the plug (7) further comprises a central through hole, wherein a transparent tube (8) is provided opened at its both ends, with graduation marks or reglette and which protrudes over the plug at least three folds the vessel (6) height, with a portion of the tube (8) entering between the vessel (6) towards the middle height of said vessel (6), traversing the plug (7); the tube and the vessel containing a translucent liquid (28) defining a top level where a marker (29) immiscible with the liquid and with different opacity floats, wherein an elongated support (9) is provided aligned with the tube (8) protrusion, arranged perpendicularly with respect to the transparent tube (8), including a light emitter (10) and a light receptor (11) facing each other, surrounding the tube (8); wherein the support (9) is mounted at an end of an elongated arm (12) which protrudes horizontally, wherein said arm (12) comprises an extension (13) including a fixed connection (13a) to a vertically arranged strap (14), wherein the extension (13) protrudes perpendicularly from the arm (12); wherein the arm (12), towards the end opposed to the support (9), is attached to a lane (15) which travels along a rail (24) comprised by a channel which is vertically arranged along the mast (3); wherein at the ends of the rail (24) attached to the mast (3), there are provided the top (25) and bottom (26) sensors for detecting the position of the lane (15); and further comprising electronic processing means (30) configured to establishing, through a logic support, an order of sequences to be treated, to record the data produced by the receptor (11) and to use said data to regulate the movement of the arm (12).

5. The device according to claim 4, wherein the strap (14) is connected to to a top (16) and bottom (17) set of pulleys.

6. The device according to claim 5, wherein the top pulley axis (16) is supported by a plate (18) comprising an L-fold attached to the rear top end of the frame mast (3).

7. The device according to claim 6, further including a motor (19) and a gearbox (19a) to which the bottomn pulley (17) is connected.

8. The device according to claim 7, comprising a cable mast (20) configured to arrange and position the connection cable (21) towards the support (9) and connects the light emitter (10) and the receptor (11).

9. The device according to claim 8, comprising an encoder (22) comprising an infrared pair (23) provided adjacent to the encoder (22), wherein said encoder (22) is coupled to the protrusion of the bottom pulley (17) axis, and wherein said encoder (22) is configured to control the movements of the arm (12).

10. The device according to claim 9, comprising a computerized terminal (31) for managing such data; printing means (32); and remote terminals (33).

11. The device according to claim 10, comprising a micro-controller configured to command the mechanical system; and wherein the motor (19) is configured to be started by relays.

12. The device according to claim 11, comprising a data storage memory.

13. The device according to claim 12, wherein the base frame (2) comprises levels and height adjustable supports.

## Patentansprüche

1. Verfahren zum Messen der volumetrischen Änderungen in einer Substanz, die Teil einer Probe (27) ist, die in einem Gefäß (6) mit geöffneter oberer Öffnung vorgesehen ist, wobei die Öffnung durch einen Stopfen (7) mit einem Loch verschlossen ist, durch das ein transparentes Rohr (8) mit offenen Enden und Teilungsstrichen verläuft, wobei das Rohr (8) sein unteres Ende innerhalb des Gefäßes (6) und sein oberes Ende außerhalb des Gefäßes (6) hat, wobei das Gefäß (6) mit einer lichtdurchlässigen Flüssigkeit (28) gefüllt ist, die eine Rohrlänge weiter füllt, bis sie ein Niveau der lichtdurchlässigen Flüssigkeit (28) bildet, wobei ein Lichtemitter-/Lichtrezeptor-Satz (10, 11) aus einem an einer Seite des Rohres (8) vorgesehenen Lichtemitter (10) und einem an der gegenüberliegenden Seite des Rohres (8) befindlichen Lichtrezeptor (11) besteht, wobei der Lichtemitter (10) und der Lichtrezeptor (11) optisch ausgerichtet sind, wobei das Verfahren die folgenden Schritte aufweist:
Bereitstellen eines Markers (29), der mit der lichtdurchlässigen Flüssigkeit (28) nicht mischbar ist und eine Opazität hat, die unterschiedlich zu der der lichtdurchlässigen Flüssigkeit ist, über dem Niveau von lichtdurchlässiger Flüssigkeit (28),
Bereitstellen des Lichtemitter-/Lichtrezeptor-Satzes (10, 11) entlang des Rohres (8) und über dem Marker (29),
Bewegen des Lichtemitter-/Lichtrezeptor- (10, 11) Satzes stromabwärts, bis der Marker (29) zwischen dem Lichtemitter (10) und dem Lichtrezeptor (11) angeordnet ist, wodurch eine Veränderung der Intensität des Lichts, das das Rohr (8) durchläuft, bestimmt wird,
Definieren einer Null("0")-Zeit und einer Anfangsposition oder Ausgangsposition an diesem Punkt,
Bewegen des Lichtemitter-/Lichtrezeptor-Satzes (10, 11) stromabwärts entlang einer bestimmten Distanz bis zu einer ersten Position, und Aufnehmen eines Höhenwerts an diesem Punkt,
Warten, bis der Marker (29), in einer stromabwärtigen Richtung, zwischen dem Lichtemitter (10) und dem Lichtrezeptor (11) an der ersten Position angeordnet ist, wodurch eine neue Veränderung der Intensität des Lichts, das den Lichtrezeptor (11) erreicht, bestimmt wird,
Aufnehmen des Höhenwerts und der Zeit, die der Marker (29) gebraucht hat, um zwischen dem Lichtemitter (10) und dem Rezeptor (11) angeordnet zu sein;
Bewegen des Lichtemitter-/Lichtrezeptor-Satzes (10, 11) stromabwärts bis zu einer zweiten Position, und Aufnehmen des neuen Höhenwerts an diesem Punkt,
Warten, bis der Marker (29), in einer stromabwärtigen Richtung, zwischen dem Lichtemitter (10) und dem Lichtrezeptor (11) angeordnet ist, wodurch eine neue Veränderung der Intensität des Lichts, das den Lichtrezeptor (11) erreicht, bestimmt wird,
Aufnehmen, an diesem Punkt, des Höhenwerts und der Zeit, die der Marker (29) gebraucht hat, um zwischen dem Lichtemitter (10) und dem Rezeptor (11) eingeordnet zu sein;
Bewegen des Lichtemitter-/Lichtrezeptor-Satzes (10, 11) stromabwärts bis zu einer dritten Position, und Aufnehmen eines neuen Höhenwerts an diesem Punkt,
Warten, bis der Marker (29), in einer stromabwärtigen Richtung, zwischen dem Lichtemitter (10) und dem Lichtrezeptor (11) angeordnet ist, und wenn der Marker (29) nicht in der Lage ist, innerhalb einer bestimmten Zeit zwischen dem Lichtemitter (10) und dem Rezeptor (11) angeordnet zu sein, Festlegen der zweiten Position als die letzte Position;
Aufnehmen der Distanz zwischen der Anfangsposition und der letzten Position, und
Aufnehmen der Zeit, die zwischen der Zeit Null "0" und der Zeit, die bis zur letzten Position akkumuliert wurde, vergangen ist.

2. Verfahren nach Anspruch 1, wobei die lichtdurchlässige Flüssigkeit (28) Wasser ist und der Marker (29) eine farbige, nicht mischbare Flüssigkeit ist.

3. Verfahren nach Anspruch 2, wobei die farbige, nicht mischbare Flüssigkeit Öl ist.

4. Vorrichtung zum Messen, mittels des Verfahrens nach einem der Ansprüche 1 bis 3, der volumetrischen Änderungen in einer Substanz, die Teil einer Probe ist, wobei die Vorrichtung einen Basisrahmen (2) aufweist, der einen Mastrahmen (3) und eine Probensammelsäule (4) aufnimmt; wobei die Probensammelsäule (4) wiederum eine Aufnahme- und Platzierungsbasis (5) aufweist, die ein Gefäß oder einen Behälter (6) trägt, der eine zu der Umgebung geöffnete obere Öffnung hat und aus einem transparenten Material gemacht ist; wobei das Gefäß (6) einen Stopfen (7) aufweist, der die obere Öffnung abdeckt und schließt; wobei der Stopfen (7) ferner ein zentrales Durchgangsloch aufweist, wobei ein an seinen beiden Enden geöffnetes transparentes Rohr (8) vorgesehen ist, mit Teilungsstrichen oder Regletten, und das über den Stopfen um mindestens die dreifache Höhe des Gefäßes (6) vorsteht, wobei ein Teil des Rohres (8) zwischen dem Gefäß (6) in Richtung der mittleren Höhe des Gefäßes (6) eintritt, den Stopfen (7) durchquerend; wobei das Rohr und das Gefäß eine lichtdurchlässige Flüssigkeit (28) enthalten, die ein oberes Niveau definiert, wo ein Marker (29), der mit der Flüssigkeit nicht mischbar ist und eine unterschiedliche Opazität hat, schwimmt, wobei ein mit dem Vorsprung des Rohres (8) ausgerichteter länglicher Träger (9) vorgesehen ist, der in Bezug auf das transparente Rohr (8) senkrecht angeordnet ist, einen Lichtemitter (10) und einen Lichtrezeptor (11) aufweisend, die einander zugewandt sind, das Rohr (8) umgebend; wobei der Träger (9) an einem Ende eines länglichen Armes (12) angebracht ist, der horizontal vorsteht, wobei der Arm (12) eine Verlängerung (13), die eine feste Verbindung (13a) zu einem vertikal angeordneten Riemen (14) hat, aufweist, wobei die Verlängerung (13) senkrecht von dem Arm (12) vorsteht; wobei der Arm (12) in Richtung zu dem Ende, das dem Träger (9) gegenüberliegt, an einer Bahn (15) angebracht ist, die entlang einer Schiene (24) läuft, die aus einem Kanal besteht, der entlang des Mastes (3) vertikal angeordnet ist; wobei an den Enden der Schiene (24), die an dem Mast (3) angebracht ist, der obere (25) und untere (26) Sensor zum Erfassen der Position der Bahn (15) vorgesehen sind; und ferner aufweisend eine elektronische Verarbeitungseinrichtung (30), die konfiguriert ist, durch logische Unterstützung eine Reihenfolge von zu behandelnden Sequenzen festzulegen, die von dem Rezeptor (11) erzeugte Daten aufzunehmen, und die Daten zu verwenden, um die Bewegung des Arms (12) zu regeln.

5. Vorrichtung nach Anspruch 4, wobei der Riemen (14) mit einem Satz aus einer oberen (16) und unteren Riemenscheibe (17) verbunden ist.

6. Vorrichtung nach Anspruch 5, wobei die Achse der oberen Riemenscheibe (16) von einer Platte (18) getragen wird, die eine an dem hinteren oberen Ende des Rahmenmastes (3) angebrachte L-Falte aufweist.

7. Vorrichtung nach Anspruch 6, ferner aufweisend einen Motor (19) und ein Getriebe (19a), mit dem die untere Riemenscheibe (17) verbunden ist.

8. Vorrichtung nach Anspruch 7, aufweisend einen Leitungsmast (20), der zum Anordnen und Positionieren des Anschlusskabels (21) in Richtung zu dem Träger (9) konfiguriert ist und den Lichtemitter (10) und den Rezeptor (11) verbindet.

9. Vorrichtung Anspruch 8, aufweisend einen Kodierer (22), der ein Infrarotpaar (23) aufweist, das benachbart zu dem Kodierer (22) vorgesehen ist, wobei der Kodierer (22) mit dem Vorsprung der Achse der unteren Riemenscheibe (17) gekoppelt ist, und wobei der Kodierer (22) konfiguriert ist, die Bewegungen des Arms (12) zu steuern.

10. Vorrichtung nach Anspruch 9, aufweisend ein computergesteuertes Endgerät (31) zum Verwalten derartiger Daten; eine Druckeinrichtung (32); und entfernte Endgeräte (33).

11. Vorrichtung nach Anspruch 10, aufweisend einen Mikrocontroller, der konfiguriert ist, das mechanische System zu befehligen; und wobei der Motor (19) konfiguriert ist, durch Relais gestartet zu werden.

12. Vorrichtung nach Anspruch 11, aufweisend einen Datenspeicher.

13. Vorrichtung nach Anspruch 12, wobei der Basisrahmen (2) Niveaus und höhenverstellbare Träger aufweist.

## Revendications

1. Procédé de mesure des changements volumétriques dans une substance qui fait partie d'un échantillon (27) ménagé au sein d'une cuve (6) avec un bec à dessus ouvert, dans lequel le bec est fermé par un bouchon (7) ayant un trou traversé par un tube transparent (8) avec des extrémités ouvertes et des traits de graduation, le tube (8) ayant son extrémité de dessous à l'intérieur de la cuve (6) et son extrémité de dessus à l'extérieur de la cuve (6), la cuve (6) étant remplie d'un liquide translucide (28) qui en outre remplit une longueur de tube jusqu'à ce qu'il forme un niveau du liquide translucide (28), un jeu émetteur/récepteur de lumière (10, 11) étant composé par un émetteur de lumière (10) ménagé d'un côté du tube (8) et un récepteur de lumière (11) de l'autre côté du tube (8), ledit émetteur de lumière (10) et ledit récepteur de lumière (11) étant alignés optiquement, le procédé comprenant l'étape de :
fourniture d'un marqueur (29) immiscible audit liquide translucide (28) et ayant une opacité différente de celle dudit liquide translucide sur ledit niveau de liquide translucide (28),
fourniture dudit jeu émetteur/récepteur de lumière (10, 11) le long du tube (8) et par-dessus ledit marqueur (29),
déplacement dudit jeu émetteur/récepteur de lumière (10, 11) en aval jusqu'à ce que ledit marqueur (29) s'interpose entre ledit émetteur de lumière (10) et ledit récepteur de lumière (11), déterminant ainsi une variation dans l'intensité de la lumière traversant le tube (8),
définition en ce point d'un temps zéro « 0 » et d'une position initiale ou position de ligne de base,
déplacement dudit jeu émetteur/récepteur de lumière (10, 11) en aval le long d'une distance spécifiée jusqu'à une première position, et enregistrement en ce point d'une valeur de hauteur,
attente que ledit marqueur (29), dans une direction aval, s'interpose entre ledit émetteur de lumière (10) et ledit récepteur de lumière (11) dans ladite première position, déterminant alors une nouvelle variation dans l'intensité de la lumière atteignant le récepteur de lumière (11),
enregistrement de ladite valeur de hauteur et du temps pris par le marqueur (29) pour s'interposer entre l'émetteur de lumière (10) et le récepteur (11) ;
déplacement dudit jeu émetteur/récepteur de lumière (10, 11) en aval jusqu'à une seconde position, et enregistrement en ce point de la nouvelle valeur de hauteur,
attente que ledit marqueur (29), dans une direction aval, s'interpose entre ledit émetteur de lumière (10) et ledit récepteur de lumière (11), déterminant ainsi une nouvelle variation dans l'intensité de la lumière atteignant le récepteur de lumière (11),
enregistrement en ce point de ladite valeur de hauteur et du temps pris par le marqueur (29) pour s'interposer entre l'émetteur de lumière (10) et le récepteur (11) ;
déplacement dudit jeu émetteur/récepteur de lumière (10, 11) en aval jusqu'à une troisième position, et enregistrement en ce point d'une nouvelle valeur de hauteur,
attente que ledit marqueur (29), dans une direction aval, s'interpose entre ledit émetteur de lumière (10) et ledit récepteur de lumière (11) et, lorsque le marqueur (29) n'est pas apte à s'interposer entre l'émetteur de lumière (10) et le récepteur (11) dans un temps spécifié, établissement de la seconde position comme la dernière position ;
enregistrement de la distance entre la position initiale et la dernière position, et
enregistrement du temps écoulé entre le temps zéro « 0 » et le temps accumulé jusqu'à la dernière position.

2. Procédé selon la revendication 1, dans lequel ledit liquide translucide (28) est de l'eau et ledit marqueur (29) est un liquide immiscible coloré.

3. Procédé selon la revendication 2, dans lequel ledit liquide immiscible coloré est de l'huile.

4. Dispositif de mesure, au moyen du procédé de l'une quelconque des revendications 1 à 3, des changements volumétriques dans une substance qui fait partie d'un échantillon, le dispositif comprenant un cadre de base (2) qui reçoit un cadre mât (3) et une colonne de collecte d'échantillon (4) ; dans lequel la colonne de collecte d'échantillon (4) comprend elle-même une base de réception et de placement (5) supportant une cuve ou un contenant (6) ayant un bec à dessus ouvert sur l'environnement et constitué d'un matériau transparent ; dans lequel ladite cuve (6) comprend un bouchon (7) qui couvre et ferme ledit bec à dessus ; dans lequel le bouchon (7) comprend en outre un trou traversant central, dans lequel un tube transparent (8) est ménagé ouvert au niveau de ses deux extrémités, avec des traits de graduation ou une réglette et qui dépasse par-dessus le bouchon au moins trois fois la hauteur de cuve (6), avec une portion de tube (8) entrant entre la cuve (6) vers la hauteur milieu de ladite cuve (6), traversant le bouchon (7) ; le tube et la cuve contenant un liquide translucide (28) définissant un niveau haut ou un marqueur (29) immiscible au liquide et avec des flotteurs d'opacité différents,
dans lequel un support allongé (9) est ménagé aligné avec la protubérance du tube (8), agencé perpendiculairement par rapport au tube transparent (8), incluant un émetteur de lumière (10) et un récepteur de lumière (11) en regard l'un de l'autre, entourant le tube (8) ; dans lequel le support (9) est monté à une extrémité d'un bras allongé (12) qui dépasse horizontalement, dans lequel ledit bras (12) comprend une extension (13) incluant un raccord fixe (13a) à une sangle agencée verticalement (14), dans lequel l'extension (13) dépasse perpendiculairement du bras (12) ; dans lequel le bras (12), vers l'extrémité opposée au support (9), est attaché à une ligne (15) qui court le long d'un rail (24) composé par un canal qui est agencé verticalement le long du mât (3) ; dans lequel aux extrémités du rail (24) attachées au mât (3), des capteurs de dessus (25) et de dessous (26) sont ménagés pour détecter la position de la ligne (15) ; et comprenant en outre des moyens de traitement électronique (30) configurés pour établir, par le biais d'un support logique, un ordre de séquences à traiter, pour enregistrer les données produites par le récepteur (11) et utiliser lesdites données pour réguler le mouvement du bras (12).

5. Dispositif selon la revendication 4, dans lequel la sangle (14) est raccordée à un jeu de dessus (16) et de dessous (17) de poulies.

6. Dispositif selon la revendication 5, dans lequel l'axe de poulie de dessus (16) est supporté par une plaque (18) comprenant un pli en L attaché à l'extrémité de dessus arrière du mât de cadre (3).

7. Dispositif selon la revendication 6, incluant en outre un moteur (19) et une boîte à engrenages (19a) à laquelle est raccordée la poulie de dessous (17).

8. Dispositif selon la revendication 7, comprenant un mât de câble (20) configuré pour agencer et positionner le câble de raccordement (21) vers le support (9) et raccorder l'émetteur de lumière (10) et le récepteur (11).

9. Dispositif selon la revendication 8, comprenant un encodeur (22) comprenant une paire à infrarouge (23) ménagée adjacente à l'encodeur (22), dans lequel ledit encodeur (22) est couplé à la protubérance de l'axe de la poulie de dessous (17), et dans lequel ledit encodeur (22) est configuré pour commander les mouvements du bras (12).

10. Dispositif selon la revendication 9, comprenant un terminal informatisé (31) destiné à gérer de telles données ; des moyens d'impression (32) ; et des bornes distantes (33).

11. Dispositif selon la revendication 10, comprenant un micro-organe de commande configuré pour commander le système mécanique ; et dans lequel le moteur (19) est configuré pour être démarré par le relais.

12. Dispositif selon la revendication 11, comprenant une mémoire de stockage de données.

13. Dispositif selon la revendication 12, dans lequel le cadre de base (2) comprend des niveaux et des supports réglables en hauteur.
